# EUROPÄISCHE PATENTSCHRIFT

(11) **EP 2 203 191 B1**
(45) Veröffentlichungstag und Bekanntmachung des Hinweises auf die Patenterteilung: **23.10.2013**
(21) Anmeldenummer: 08837018.4
(22) Anmeldetag: 30.09.2008
(51) Int. Cl.: A61L 2/20, B67C 7/00

(54) **VORRICHTUNG ZUM STERILISIEREN VON BEHÄLTNISSEN**
DEVICE FOR STERILIZING CONTAINERS
DISPOSITIF POUR STÉRILISER DES RÉCIPIENTS

(30) Priorität: 02.10.2007 DE 102007047259
(43) Veröffentlichungstag der Anmeldung: 07.07.2010
(73) Patentinhaber: Krones AG, 93073 Neutraubling (DE)
(72) Erfinder: LAUMER, Roland, 93047 Regensburg (DE); SCHMID, Manfred, 93089 Aufhausen (DE); BERTHOLD, Burgmeier, 89561 Dischingen/Eglingen (DE)
(74) Vertreter: Hannke, Christian
(86) Internationale Anmeldenummer: PCT/EP2008/063090
(87) Internationale Veröffentlichungsnummer: WO 2009/047171

(56) Entgegenhaltungen:
- EP-A- 1 601 606
- EP-A- 1 821 010
- GB-A- 407 672

## Beschreibung

Die vorliegende Erfindung bezieht sich auf eine Vorrichtung zum Sterilisieren von Gefäßen, und insbesondere von Kunststoffflaschen, gemäß dem Oberbegriff des Anspruchs 1 und wie aus der EP 1 821 000 bekannt. Es wird jedoch darauf hingewiesen, dass die vorliegende Erfindung auch auf andere Behandlungen von Behältnissen anwendbar ist, wie beispielsweise das Verschließen, oder auch das Herstellen oder Reinigen der Behältnisse.

Aus dem Stand der Technik ist eine Vielzahl von derartigen Vorrichtungen bekannt. Dabei ist es unter anderem bekannt, die Gefäße mit einem Wasserstoffperoxidgas zu desinfizieren. Bei einer aus dem Stand der Technik bekannten Vorrichtung werden dabei die Gefäße mit einem kreisförmigen Transportkarussell durch einen Sterilisationsraum geführt und innerhalb dieses Sterilisationsraums mit einem Wasserstoffperoxid enthaltenden Gas desinfiziert. Bei derartigen Vorrichtungen tritt das Problem auf, dass ein Austreten dieses Wasserstoffperoxid enthaltenden Gases möglichst effizient zu verhindern ist, da derartige Gase schädlich für den Benutzer sind.

Die vorliegende Erfindung ist sowohl für Vorrichtungen anwendbar, die eine Sterilisation der Innenwandung der Gefäße bewirken, als auch für solche Vorrichtungen, die eine Sterilisation der Außenwandung der Gefäße durchführen. Des Weiteren ist die Erfindung auch für Vorrichtungen geeignet, welche die Gefäße in steriler Umgebung mit einem Getränk befüllen.

Im Stand der Technik ist es dabei bekannt, sogenannte Wasserschlösser zu verwenden, um einen Austritt des Gases aus der Vorrichtung zu verhindern. Dabei wird in einem stehenden Behältnis eine Flüssigkeit geführt und ein Element des drehenden Transportkarussells, welches beispielsweise auch die Gefäße führt, gleitet permanent durch diese Flüssigkeit. Auf diese Weise wird einerseits erreicht, dass eine Drehung möglich ist und andererseits kann das Gas nicht an dieser Flüssigkeitsbarriere entweichen. Mit anderen Worten bildet dieses Wasserschloß eine drehbare dichte Verbindung bzw. ein eine hydraulische Dichtung bildendes Flüssigkeitsbad.

Aus der DE 2 139 057 ist ein Apparat zur septischen Konditionierung beliebiger Produkte bekannt. Dieser Apparat weist einen gegen die äußere Atmosphäre abgedichteten Raum auf, der durch eine Kuppel abgedichtet wird. Weiterhin ist eine drehbare dichte Verbindung mit einem eine hydraulische Verbindung bildenden Flüssigkeitsbad vorgesehen.

Aus der EP 1 601 606 B1 ist eine Maschine zur aseptischen Behandlung von Behältern in einer Abfüllanlage bekannt. Auch hier sind zwei mit Flüssigkeit gefüllte ringförmige Behältnisse vorgesehen, in denen jeweils ringförmige Körper gleiten, um auf diese Weise eine Abdichtung zu erreichen. Die beiden mit Flüssigkeit gefüllten Behältnisse schließen sich dabei direkt an den sterilen Raum an.

Der vorliegenden Erfindung liegt die Aufgabe zugrunde, die Sicherheit von derartigen Vorrichtungen hinsichtlich des Austritts des sterilisierenden Mediums zu vergrößern. Eine weitere Aufgabe besteht darin, die aus dem Stand der Technik bekannten Vorrichtungen einfacher zu gestalten. Dies wird durch eine Vorrichtung nach Anspruch 1 erreicht. Vorteilhafte Ausführungsformen und Weiterbildungen sind Gegenstand der Unteransprüche.

Eine erfindungsgemäße Vorrichtung zum Sterilisieren von Gefäßen weist einen Behandlungsraum auf, der mit einem sterilisierenden gasförmigen Medium unter vorgegebenen thermodynamischen Bedingungen und insbesondere mit einem vorgegebenen Druck befüllt ist, ein mit einem flüssigen Medium gefülltes Behältnis, welches sich entlang einer geschlossenen Bahn erstreckt sowie einer Vielzahl von Halteinrichtungen zum Halten der Gefäße, wobei die Halteeinrichtungen drehbar gegenüber wenigstens einer der den Behandlungsraum bildenden Wände angeordnet sind. Weiterhin ist eine Trennwand vorgesehen, die abschnittsweise in das in dem Behältnis befindliche flüssige Medium hineinragt und gegenüber dem Behältnis drehbar angeordnet ist. Die Trennwand ist dabei insbesondere drehfest mit den Halteeinrichtungen verbunden. Erfindungsgemäß ist zwischen dem Behandlungsraum und dem in dem Behältnis befindlichen flüssigen Medium eine Abführöffnung vorgesehen, über die das gasförmige Medium abführbar ist.

Dabei ist es einerseits möglich, dass die Abführöffnung außerhalb der Aussenwandung des Behältnisses angeordnet ist. Es wäre jedoch auch möglich, dass die Abführöffnung oberhalb eines Füllstandes des in dem Behältnis befindlichen flüssigen Mediums angeordnet (und daher möglicherweise noch innerhalb des Behältnisses bzw. innerhalb des von den Wandungen des Behältnisses gebildeten Hohlraums) ist.

Bei beiden Gestaltungen wäre daher definitionsgemäß die Abführöffnung zwischen dem Behältnis und dem Behandlungsraum vorgesehen. Der Begriff "zwischen" wird daher unter Bezugnahme auf den Raum verstanden, der zwischen dem Behältnis und insbesondere der in diesem Behältnis befindlichen Flüssigkeit und dem Behandlungsraum liegt.

Bei dem sterilisierenden Medium handelt es sich vorzugsweise um Wasserstoffperoxid oder ein Wasserstoffperoxid enthaltendes Gas. Bei dem flüssigen Medium handelt es sich insbesondere um Wasser und besonders bevorzugt um eine bakteriostatische Lösung. Die Trennwand ragt vorzugsweise derart in das flüssige Medium hinein, dass das flüssige Medium gemeinsam mit der Trennwand wie eine Dichtung wirkt, welche den Behandlungsraum gegenüber der Umgebung abdichtet. Die Kombination aus dem Behältnis mit dem flüssigen Medium und der Trennwand bildet das oben erwähnte "Wasserschloß".

Vorzugsweise ist das Behältnis mit der Flüssigkeit im Wesentlichen kreisförmig ausgebildet und der Behandlungsraum ist damit insbesondere ein steriler Behandlungsraum.

Durch die erfindungsgemäße Abführöffnung kann ein Druckausgleich zwischen dem Wasserschloß und dem Behandlungsraum gebildet werden und damit kann in besonders sicherer Weise ein unerwünschtes, das heißt anderweitig austretendes gasförmiges Medium, vermieden werden.

Vorzugsweise ist zwischen dem Behandlungsraum und dem in dem Behältnis befindlichen flüssigen Medium ein Pufferraum für das gasförmige Medium vorgesehen. Es wird darauf hingewiesen, dass es auch möglich wäre, lediglich den Pufferraum, und nicht die oben erwähnte Abführöffnung vorzusehen. Die Anmelderin behält sich vor, auch einen entsprechenden Anspruch auf eine Vorrichtung mit dem erwähnten Pufferraum zu richten. Vorzugsweise steht das gasförmige Medium in dem Pufferraum unter einem geringeren Druck als in dem Behandlungsraum. Besonders bevorzugt ist jedoch der Druck, unter dem das Behältnis in dem Pufferraum steht, größer als der (atmosphärische) Außendruck.

Durch dieses Druckgefälle zwischen dem Behandlungsraum und dem Pufferraum kann erreicht werden, dass das gasförmige Medium stets aus dem Behandlungsraum heraus in den Pufferrum gelangt. Auf diese Weise kann der Behandlungsraum stets als steriler Raum beibehalten werden. Der Pufferraum hingegen ist bevorzugt nicht als steriler Raum ausgeführt.

Vorzugsweise ist die Trennwand an einer Abdeckung angeordnet, die den Behandlungsraum abdeckt. Genauer gesagt, sind auch die Halteeinrichtungen für die Behältnisse drehfest gegenüber dieser Abdeckung angeordnet.

Bei einer weiteren vorteilhaften Ausführungsform ist der Behandlungsraum von einer stehenden Seitenwand und einer sich gegenüber der stehenden Seitenwand drehenden Seitenwand begrenzt. Diese Ausgestaltung erlaubt eine besonders effiziente Abdichtung des sterilen Behandlungsraums. Vorzugsweise weist die Vorrichtung zwei Behältnisse der oben beschriebenen Art auf, in die jeweils die oben erwähnten Trennwände hineinragen. Dabei sind vorzugsweise beide Behältnisse jeweils stehend angeordnet und die beiden Trennwände drehend gegenüber diesen Behältnissen. Bei einer weiteren vorteilhaften Ausführungsform sind die Trennwände drehfest mit der oben erwähnten Abdeckung verbunden.

Bei einer weiteren vorteilhaften Ausführungsform ist eines dieser Behältnisse unterhalb der zu sterilisierenden Gefäße bzw. deren Trägereinrichtungen und das andere Behältnis oberhalb der zu sterilisierenden Gefäße angeordnet. Auf diese Weise kann eine vollständige Abdichtung des Behandlungsraums erreicht werden. Dabei ist es möglich, bei beiden mit dem flüssigen Medium befüllten Behältnissen jeweils Pufferräume der oben beschriebenen Art vorzusehen sowie auch in beiden Fällen Abführöffnungen, über die das gasförmige Medium abführbar ist.

Vorzugsweise weist das Behältnis eine radial äußere Begrenzungswand und eine radial innere Begrenzungswand auf und die radial äußere Begrenzungswand ist höher als die radial innere Begrenzungswand. Auf diese Weise wird erreicht, dass zu Reinigungszwecken das Behältnis so befüllt werden kann, dass das Reinigungsmittel bezüglich des Behältnisses nach innen fließt. Bei den Behältnissen handelt es sich insbesondere im ringsegment oder ringförmig ausgebildete Kanäle.

Bei einer weiteren vorteilhaften Ausführungsform sind zwischen dem Behandlungsraum und dem Pufferraum wenigstens zwei Umlenkwände vorgesehen, welche eine Strömungsrichtung des gasförmigen Mediums in Richtung der Abführöffnung umlenken. Durch den in dem Pufferraum stehenden niedrigeren Druck strömt das gasförmige Medium von dem Behandlungsraum in den Pufferraum und wird dabei durch die Umlenkwände umgelenkt. Dabei ist bevorzugt eine Umlenkwand stationär und die andere Umlenkwand drehbar gegenüber dieser stationären Umlenkwand angeordnet.

Vorzugsweise ist die Abdeckung drehbar angeordnet.

Bei einer weiteren vorteilhaften Ausführungsform ist an der Abdeckung wenigstens eine Düse zur Reinigung des Pufferraums vorgesehen. Vorzugsweise ist eine Vielzahl von Düsen in Umfangsrichtung und besonders vorteilhaft auch in radialer Richtung verteilt. Bei einer weiteren vorteilhaften Ausführungsform ist auch eine Düse zur Reinigung des Zwischenraums zwischen den wenigstens zwei Umlenkwänden vorgesehen.

Vorteilhaft ist auch an dem Behältnis wenigstens eine Düse zur Reinigung des Behältnisses vorgesehen. Damit ist auch möglich, das oben erwähnte "Wasserschloß" zu reinigen. Bevorzugt dient diese Düse auch zum Befüllen dieses Behältnisses.

Bei einer weiteren vorteilhaften Ausführungsform ist in dem Pufferraum auch wenigstens eine stationär angeordnete Düse vorgesehen. Bevorzugt ist diese Düse in einem Bodenabschnitt des Pufferraums angeordnet.

Vorzugsweise ist das Behältnis mit der Flüssigkeit unterhalb der zu sterilisierenden Gefäße angeordnet. Es wäre jedoch auch möglich, dass das Behältnis oberhalb der zu sterilisierenden Gefäße vorgesehen ist, oder, dass wie ausgeführt, zwei derartige Behältnisse vorgesehen sind.

Bei einer weiteren vorteilhaften Ausführungsform ist die Abführöffnung oberhalb eines Füllniveaus des flüssigen Mediums angeordnet. Besonders bevorzugt schließt sich hierbei an die Abführöffnung ein Abführrohr für das gasförmige Medium an, welches sich abschnittsweise durch das Behältnis hindurch erstreckt. Vorteilhaft erstreckt sich das Abführrohr wenigstens abschnittsweise durch die in dem Behältnis befindliche Flüssigkeit.

Durch diese Ausführungsform kann auf einen zusätzlichen Pufferraum, der zwischen der Aussenwandung des Behältnisses und dem Behandlungsraum gebildet wird, verzichtet werden. Der Pufferraum wird hier bevorzugt oberhalb des flüssigen Mediums gebildet.

Weitere Vorteile und Ausführungsformen ergeben sich aus den beigefügten Zeichnungen:

Darin zeigen:
- Fig. 1: eine schematische Darstellung einer Vorrichtung zum Sterilisieren von Gefäßen;
- Fig. 2: eine Teilansicht einer erfindungsgemäßen Vorrichtung zum Sterilisieren von Gefäßen;
- Fig. 3: eine weitere Teilansicht einer erfindungsgemäßen Vorrichtung zum Sterilisieren von Gefäßen
- Fig. 4: eine Detailansicht einer oberen Abdichteinrichtung;
- Fig. 5: eine detaillierte Darstellung einer unteren Abdichteinrichtung;
- Fig. 6: eine weitere Detailansicht einer erfindungsgemäßen Vorrichtung;
- Fig. 7: eine Detailansicht einer erfindungsgemäßen Vorrichtung in einer weiteren Ausführungsform; und
- Fig. 8: eine Draufsicht auf die Vorrichtung aus Fig. 7.

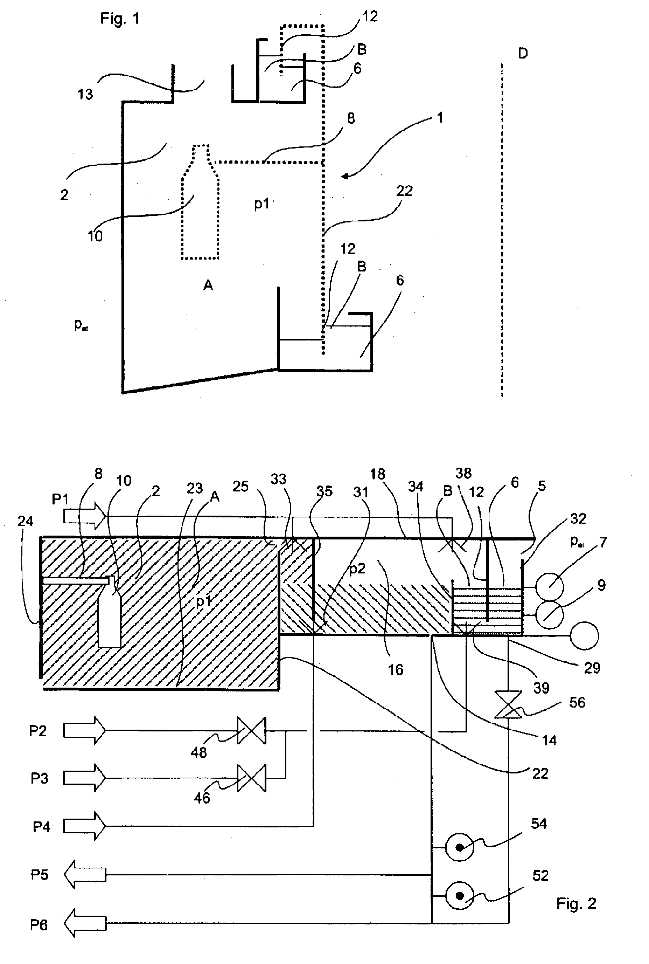

Fig. 1 zeigt eine Vorrichtung 1 zum Sterilisieren von Gefäßen. Diese Vorrichtung weist einen Behandlungsraum 2 auf, durch den hindurch Gefäße 10 geführt werden, um diese zu sterilisieren. Dieser Behandlungsraum 2 wird teilweise durch stationäre Komponenten und teilweise durch sich gegenüber diesen stationären Komponenten drehende Komponenten gebildet. In die Fig. 1 sind die stationären Komponenten durch durchgezogene Linien dargestellt und die sich diesen gegenüber drehenden Komponenten durch punktierte Linien.

In den Behandlungsraum 2 wird ein steriles Gas über eine Einführöffnung 13 eingeführt. Um die drehenden Komponenten gegenüber den stehenden Komponenten abzudichten, sind zwei Behältnisse 6 vorgesehen, die jeweils mit einer bakteriostatischen Lösung oder beispielsweise mit Wasser befüllt sind. Diese Lösung ist mit dem Bezugszeichen B gekennzeichnet. Jeweils eine Trennwand 12 ragt dabei sowohl in das untere Behältnis 6 als auch das obere Behältnis 6 und zwar derart, dass diese Trennwand 12 in die Flüssigkeit B eintaucht. Durch das Eindringen dieser Trennwand in die beiden Behältnisse 6 wird insgesamt der Behandlungsraum 2 abgedichtet. Das Bezugszeichen 8 bezieht sich auf eine Trägereinrichtung, welche die Gefäße 10 durch den Behandlungsraum 2 führt.

Innerhalb des Behandlungsraums herrscht ein erster Druck p1 und außerhalb des Behandlungsraums ein atmosphärischer Druck Pₐₜ, der geringer ist als der Druck p1.

Durch diese Druckunterschiede ergibt sich, dass sich die Füllstände des flüssigen Mediums B nicht im Gleichgewicht befinden, sondern das Flüssigkeitsniveau jeweils auf derjenigen Seite der Trennwand 12, die in den Behandlungsraum 2 hineinragt, niedriger ist als auf derjenigen Seite der Trennwand 12, die außerhalb des Behandlungsraums 2 liegt. Die drehbaren Einheiten der Vorrichtung 1 sind dabei um eine Drehachse D drehbar. Die beiden Behältnisse 6 erstrecken sich ring(segment)förmig ebenfalls um diese Drehachse D. Auch der Behandlungsraum 2 erstreckt sich kreisring(segment)förmig um die Drehachse D.

Fig. 2 zeigt eine Detailansicht einer erfindungsgemäßen Vorrichtung zum Sterilisieren von Gefäßen. Auch hier werden die Behältnisse 10 zumindest abschnittsweise entlang einer Kreisbahn durch einen Behandlungsraum 2 geführt. Dabei sind die Gefäße 10 an Halteeinrichtungen 8 angeordnet. Die Halteeinrichtungen 8 sind wiederum an einer Seitenwand 24, welche den Behandlungsraum 2 begrenzt, befestigt. In dem Behandlungsraum 2 ist während des laufenden Betriebs ein steriles Medium A vorgesehen. In Fig. 2 ist lediglich ein Behältnis 6, welches mit der Lösung B befüllt ist, dargestellt. Ein weiteres Behältnis (nicht gezeigt) dient dazu, um den Raum zwischen der Wand 24 und dem (stationären) Boden 23 abzudichten.

Das Bezugszeichen 18 kennzeichnet die Abdeckung, welche den Behandlungsraum 2 abdeckt. An dieser Abdeckung 18 ist auch eine Trennwand 12 angeordnet, die, wie oben dargestellt, in das Behältnis 6 und die Lösung B hineinragt. Zwischen dem Behandlungsraum und dem Behältnis 6 ist eine Abführöffnung 14 vorgesehen. Über diese Abführöffnung 14 kann im laufenden Betrieb das gasförmige Medium abgeführt werden. Das Bezugszeichen 16 bezieht sich auf einen Pufferraum für das gasförmige Medium A. Durch die Abführöffnung 14 wird erreicht, dass innerhalb dieses Pufferraums 16 ein Druck p2 herrscht, der geringer ist als der Druck p1 in dem Behandlungsraum 2, jedoch größer als der atmosphärische Druck pₐₜ außerhalb der Vorrichtung. Das Bezugszeichen 35 bezieht sich auf eine Umlenkwand, die damit ein Labyrinth bildet, entlang dessen der in dem Behandlungsraum 2 bestehende Druck p1 zu dem in dem Pufferraum 16 bestehenden Druck p2 abgebaut werden kann.

Das Bezugszeichen 22 kennzeichnet eine weitere (stehende) Seitenwand, die den Behandlungsraum 2 begrenzt. Damit wird der Behandlungsraum 2 durch die beiden Seitenwände 22 und 24, den Boden 23 und die Abdeckung 18 begrenzt. Zwischen der Abdeckung 18 und der Seitenwand 22 wird eine Öffnung bzw. ein Spalt 25 gebildet, durch den das gasförmige Medium A aus dem Behandlungsraum 2 heraus gelangt. An diesem (Ring)spalt 25 entsteht die höchste Strömungsgeschwindigkeit des gasförmigen Mediums. Daher kann deine Sterilgrenze zwischen Reinraum und Umgebung auf diesen Ringspalt gelegt werden.

Das Bezugszeichen 5 bezieht sich auf einen Ablauf für insbesondere Luft, um einen Druckausgleich zwischen dem Pufferraum 16 und dem Außenraum der Vorrichtung zuzulassen. Die Bezugszeichen 7 und 9 beziehen sich auf Füllstandsbestimmungseinrichtungen, welche einen Füllstand des flüssigen Mediums B innerhalb des Behältnisses 6 bestimmen. Auch ist es möglich, dass in Reaktion auf einen zu niedrigen Füllstand das Behältnis wieder befüllt werden kann. Das Behältnis 6 wird durch eine erste Begrenzungswand 32 und eine zweite Begrenzungswand 34 in radialer Richtung begrenzt. Dabei ist die innere Begrenzungswand 34 niedriger als die äußere Begrenzungswand 32. Dies hat den Vorteil, dass das Behältnis 6 derart befüllt werden kann, dass das flüssige Medium B in den Pufferraum 16 gelangt, um auch diesen reinigen zu können.

Die Bezugszeichen 31, 33, und 38 beziehen sich auf Düsen, die zur Innenreinigung der Vorrichtung 1 dienen. Dabei sind die beiden Düsen 33 und 38 an der drehbaren Abdeckung 18 angeordnet und reinigen, wie in Fig. 2 gezeigt, den Bereich links bzw. rechts bezüglich der Umlenkwand 35. Die Düse 31 ist stationär angeordnet und dient zur Innenreinigung des Pufferraums 16. Weiterhin ist in dem Behältnis 6 eine Düse 39 zur Innenreinigung desselben vorgesehen. Das Bezugszeichen 29 bezieht sich auf eine Ablassöffnung, über welche aus dem Behältnis das flüssige Medium B abgelassen werden kann. Die unteren Düsen 31, 39 reinigen damit den stehenden Teil der Ringkanäle und die Düsen 33 und 38 deren drehenden Teil.

Die Bezugszeichen 46, 48 und 56 beziehen sich auf unterschiedliche Ventile, deren Funktion im Folgenden genauer erklärt wird. Im laufenden Betrieb sind die beiden Ventile 46 und 48 geschlossen. Über einen nicht gezeigten Einlass gelangt das gasförmige Medium in den Behandlungsraum 2 und wird in definierter Weise über die Abführöffnung 14 entlang des Pfeils P6 abgeführt. Die Bezugszeichen 52 und 54 beziehen sich auf Blenden, die als Wasserabscheider dienen, um gegebenenfalls Flüssigkeit, welche über die Abführöffnung 14 gelangt, abführen zu können.

Falls der Füllstand des flüssigen Mediums B unter ein gewisses Niveau sinkt, kann das Ventil 48 geöffnet und das Behältnis 6 wieder über die Düse 39 mit dem flüssigen Medium B befüllt werden. Während der sog. CIP - Reinigung (cleaning in place) gelangt ein Reinigungsmittel entlang des Pfeils P1 über die Düse 33 in den zweiten Pufferraum 16, um dessen Innenraum zu reinigen. Ebenso gelangt entlang des Pfeils P2 über die Düse 39 das Reinigungsmittel in das Behältnis 6 sowie entlang des Pfeils P4 über die Düse 31 ebenfalls in den Pufferraum 16. Anschließend an diese Reinigung kann auch wiederum das Behältnis 6 mit dem flüssigen Medium befüllt werden.

Bei der in Fig. 1 gezeigten Ausführungsform sind die unterhalb des Behandlungsraums 2 vorgesehenen Ventile stationär angeordnet.

Weiterhin ist es auch möglich, das Ventil 56 zu öffnen und aus dem Behältnis 6 das flüssige Medium bzw. auch das Reinigungsmittel abzuziehen. Entlang des Pfeils P4 kann Wasserstoffperoxid bzw. ein Wasserstoffperoxid enthaltendens Gemisch in den Pufferraum 16 eingeleitet werden.

Zur SIP - Reinigung (sterilisation in place) kann der Pufferraum 16 wiederum über die Düsen 31, 33 und 38 mit einem Sterilisationsmedium befüllt und gleichzeitig über die Abführöffnung 14 dieses Medium wieder abgezogen werden. In dem Pufferraum 16 befindet sich nicht steriles gasförmiges Medium und, wie oben erwähnt, im Behandlungsraum 2 das sterile Medium A.

Bei der in Fig. 2 gezeigten Ausführungsform könnten auch zwei Behältnisse 6 nebeneinander (bzw. konzentrisch zueinander) angeordnet sein. Genauer gesagt, könnte zusätzlich zu dem in Fig. 2 gezeigten Behältnis 6 noch ein innen liegendes zweites Behältnis mit einer entsprechenden Trennwand 12 vorgsehen sein. Dieses innenliegende Behältnis könnte dabei mit einem sterilisierenden Medium gefüllt sein und den Behandlungsraum 2 gegenüber dem Pufferraum 16 abdichten, wohingegen das in

Fig. 2 gezeigte Behältnis mit Wasser gefüllt sein könnte, um den Pufferraum 16 gegenüber der Umgebung abzudichten. Auf diese Weise könnte ein Austritt von aggressiven Gasen aus dem Pufferraum noch effizienter verhindert werden. Auch könnte durch derart redundante Dichtungen insgesamt auch noch bei Ausfall einer Dichtung der Dichtungseffekt beibehalten werden. Auch.in den weiteren im Rahmen dieser Offenbarung gezeigten Ausführungsformen könnten zwei Behältnisse nebeneinander vorgesehen sein.

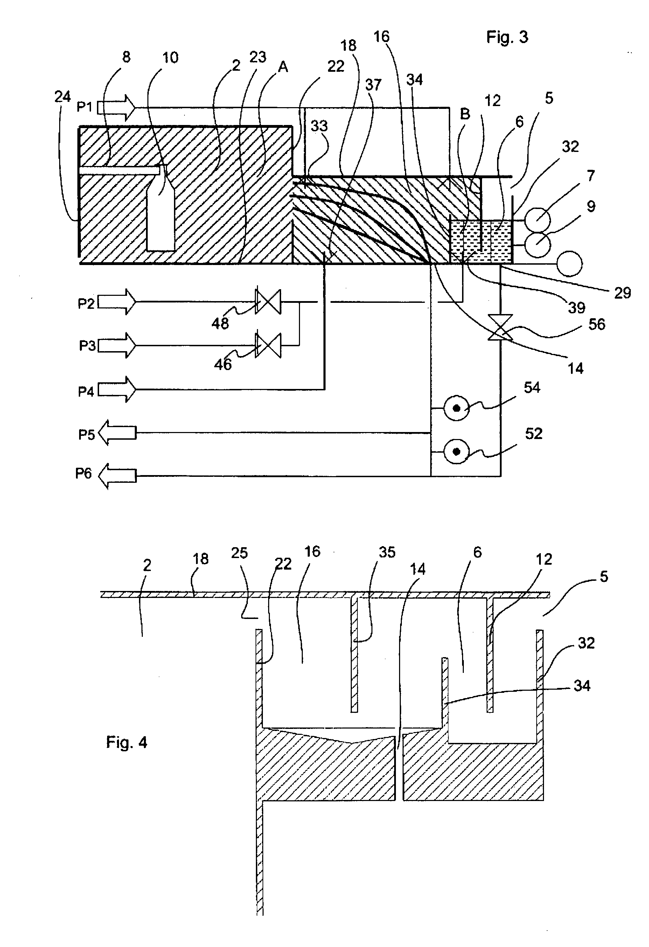

Fig. 3 zeigt eine schematische Darstellung einer unteren Dichtung, das heißt einer Dichtung, die unterhalb der zu sterilisierenden Gefäße angeordnet ist. Im Gegensatz zu der in Fig. 2 gezeigten Dichtung wird bei der in Fig. 3 gezeigten Dichtung zwischen dem Behandlungsraum 2 und dem Pufferraum 16 die Strömung des Mediums nicht mehrfach umgelenkt. Hiervon abgesehen entspricht die Anordnung der unteren Dichtung weitgehend der Anordnung für die obere Dichtung und wird daher nicht mehr im Detail erläutert. Das gasförmige Medium A gelangt entlang der durchgezogenen Linien von dem Behandlungsraum 2 in Richtung der Abführöffnung 14.

Fig. 4 zeigt eine detaillierte Ansicht einer oberen Dichtung. Dabei ist insbesondere auch das Behältnis 6 bzw. das Wasserschloß erkennbar und die in Fig. links bezüglich des Behältnisses 6 angeordnete Wasserstoffperoxidbarriere bzw. der Pufferraum 16 mit der Abführöffnung 14.

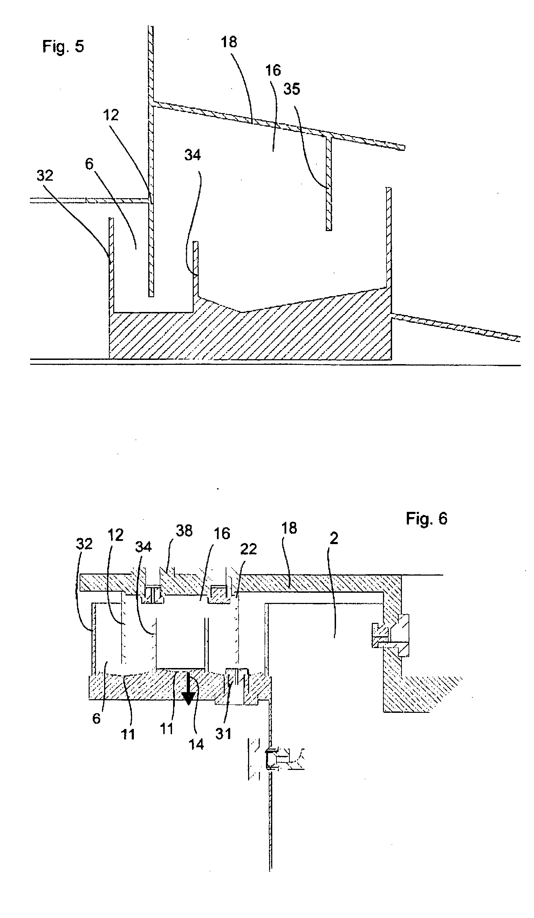

Fig. 5 zeigt eine Darstellung für die untere Dichtung. Auch in dieser unteren Dichtung kann in einem Pufferraum 16 eine (nicht gezeigte) Abführöffnung für das gasförmige Medium A vorgesehen sein.

Fig. 6 zeigt eine detailliertere Darstellung einer oberen Abdichtung. Man erkennt hier Einsenkungen bzw. Mulden 11, die insbesondere zum Sammeln von Flüssigkeit von Reinigungsflüssigkeit dienen. Das Bezugszeichen 31 zeigt auch hier eine Düse, die zum Einführen eines Mediums in den Pufferraum 16 dient. Der Pfeil P8 veranschaulicht das über die Abführöffnung im laufenden Betrieb austretende gasförmige Medium A.

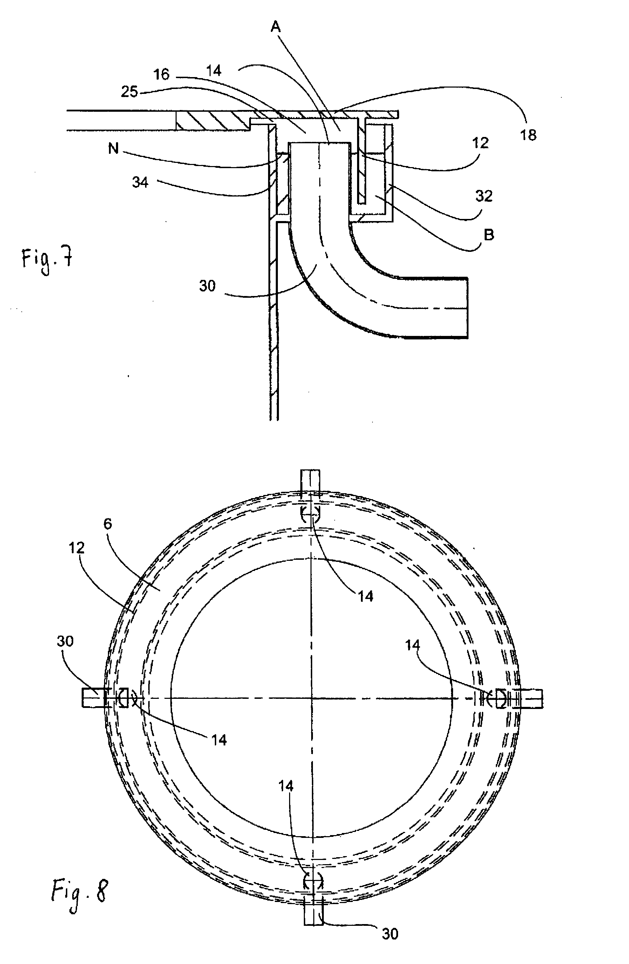

Fig. 7 zeigt eine Detailansicht einer weiteren Ausführungsform der erfindungsgemäßen Vorrichtung. Während bei den in den vorangegangenen Figuren gezeigten Ausführungsformen die Abführöffnung 14 außerhalb des Behältnisses 6 bzw. zwischen der Wand 34 des Behältnisses 6 und dem Behandlungsraum 2 angeordnet ist, ist sie bei der in Fig. 7 gezeigten Ausführungsform zwischen den beiden Wänden 34 und 32, jedoch über einem Befüllungsniveau N des Behältnisses 6 vorgesehen. Genauer gesagt ist die Abführöffnung 14 zwischen der Trennwand 12 und der Wand 34 vorgesehen.

Der Pufferraum 16 ist hier oberhalb des flüssigen Mediums B auf der linken Seite bzgl. der Trennwand 12. Das Bezugszeichen 30 bezieht sich auf ein Abführrohr, an dessen Ende sich die Abführöffnung 14 befindet. Dieses Abführrohr 30 erstreckt sich ausgehend von einem Boden 27 des Behältnisses 6 im Wesentlichen vertikal nach oben und damit durch die Flüssigkeit B hindurch. Unterhalb des Bodens 27 schließt sich ein gekrümmter Abschnitt des Rohres 30 an.

Bei der in Fig. 7 gezeigten Ausführungform ist die Abführöffnung 14 niedriger angeordnet als die oberen Enden der beiden Wände 32, 34. Auf diese Weise ist es möglich, dass überschüssige Flüssigkeit über die Abführöffnung 14 weggeführt wird. Es wäre jedoch auch möglich, einen (nicht gezeigten) Verschlussmechanismus (beispielsweise unter Verwendung eines Schwimmers) vorzusehen, der bewirkt, dass die Abführöffnung geschlossen wird, wenn das Befüllungsniveau N bis zur Höhe der Abführöffnung 14 ansteigt.

Fig. 7 zeigt damit eine besonders platzsparende Ausführungsform. Oberhalb des Flüsigkeitsniveaus N entsteht ein weiterer Ringkanal, in dem ein Unterdruck herrscht. Auch hier entsteht an dem (Ring)spalt 25 die höchste Strömungsgeschwindigkeit.

Fig. 8 zeigt eine Draufsicht auf eine Vorrichtung aus Fig. 7. Man erkennt, dass hier insgesamt vier Abführöffnungen 14 innerhalb des Behältnisses 6 angeorndet sind. Diese vier Abführöffnungen 14 sind dabei gleichmäßig in Umfangsrichtung verteilt. Die Abführöffnungen 14 weisen dabei einen Querschnitt auf, der geringfügig kleiner ist als der radiale Abstand zwischen der Trennwand 12 und der Wand 34 des Behältnisses 6.

Das gesamte Dichtungssystem wird vorteilhaft zwischen einer Trägerplatte der Vorrichtung und einer Einhausung des Reinraums eingesetzt.

## Patentansprüche

1. Vorrichtung zum Sterilisieren von Gefäßen (10) mit einem Behandlungsraum (2), der mit einem sterilisierenden gasförmigen Medium (A) unter vorgegebenen thermodynamischen Bedingungen befüllt ist, einem mit einem flüssigen Medium (B) gefüllten Behältnis (6), welches sich entlang einer geschlossenen Bahn erstreckt, mit einer Vielzahl von Halteeinrichtungen (8)
zum Halten der Gefäße, wobei die Halteeinrichtungen (8) drehbar gegenüber wenigstens einer der den Behandlungsraum (2) bildenden Wände (18, 22, 23, 24) angeordnet sind, mit einer Trennwand (12), die in das in dem Behältnis (6) befindliche flüssige Medium (B) hineinragt und gegenüber dem Behältnis (6) drehbar angeordnet ist
**dadurch gekennzeichnet, dass**
zwischen dem Behandlungsraum (2) und dem in dem Behältnis (6) befindlichen flüssigen Medium (B) eine Abführöffnung (14) vorgesehen ist, über die das gasförmige Medium (A) abführbar ist.

2. Vorrichtung nach Anspruch 1,
**dadurch gekennzeichnet, dass**
zwischen dem Behandlungsraum (2) und dem in dem Behältnis (6) befindlichen flüssigen Medium (B) ein Pufferraum (16) für das gasförmige Medium (A) vorgesehen ist.

3. Vorrichtung nach Anspruch 2,
**dadurch gekennzeichnet, dass**
das Medium (A) in dem Pufferraum (16) unter einem geringeren Druck (p2) steht als in dem Behandlungsraum (2).

4. Vorrichtung (1) nach wenigstens einem der vorangegangenen Ansprüche,
**dadurch gekennzeichnet, dass**
die Trennwand an einer Abdeckung (18) angeordnet ist, die den Behandlungsraum (2) abdeckt.

5. Vorrichtung (1) nach wenigstens einem der vorangegangenen Ansprüche,
**dadurch gekennzeichnet, dass**
der Behandlungsraum (2) von einer stehenden Seitenwand (22) und einer sich gegenüber der stehenden Seitenwand (22) drehenden Seitenwand (24) begrenzt wird.

6. Vorrichtung (1) nach wenigstens einem der vorangegangenen Ansprüche,
**dadurch gekennzeichnet, dass**
die Vorrichtung (1) zwei voneinander getrennte Behältnisse (6) aufweist sowie zwei Trennwände (12), die jeweils in die Behältnisse (6) hineinragen.

7. Vorrichtung (1) nach wenigstens einem der vorangegangenen Ansprüche,
**dadurch gekennzeichnet, dass**
das Behältnis (6) eine radial äußere Begrenzungswand (32) und eine radial innere Begrenzungswand (34) aufweist und die radial äußere Begrenzungswand (32) niedriger ist als die radial innere Begrenzungswand (34)

8. Vorrichtung (1) nach wenigstens einem der Ansprüche 2, 3,
**dadurch gekennzeichnet, dass** zwischen dem Behandlungsraum (2) und dem Pufferraum (16) wenigstens zwei Umlenkwände (34,36) vorgesehen sind, welche eine Strömungsrichtung des gasförmigen Medium (A) in Richtung der Abführöffnung umlenken.

9. Vorrichtung (1) nach Anspruch 4,
**dadurch gekennzeichnet, dass** die Abdeckung (18) drehbar angeordnet ist.

10. Vorrichtung (1) nach Anspruch 9,
**dadurch gekennzeichnet, dass** an der Abdeckung (18) wenigstens eine Düse (38) zur Reinigung des Pufferraums (16) vorgesehen ist.

11. Vorrichtung (1) nach wenigstens einem der vorangegangenen Ansprüche,
**dadurch gekennzeichnet, dass** in dem Behältnis (6) wenigstens eine Düse (39) zur Reinigung des Behältnisses (6) vorgesehen ist.

12. Vorrichtung (1) nach wenigstens einem der vorangegangenen Ansprüche 2, 3, 8
**dadurch gekennzeichnet, dass** in dem Pufferraum (16) wenigstens eine stationär angeordnete Düse (37) vorgesehen ist.

13. Vorrichtung (1) nach wenigstens einem der vorangegangenen Ansprüche,
**dadurch gekennzeichnet, dass** das Behältnis (6) unterhalb der zu sterilisierenden Gefäße (10) angeordnet ist.

14. Vorrichtung (1) nach wenigstens einem der vorangegangenen Ansprüche,
**dadurch gekennzeichnet, dass** die Abführöffnung (14) oberhalb eines Füllniveaus (N) des flüssigen Mediums (B) angeordnet ist.

15. Vorrichtung (1) nach wenigstens einem der vorangegangenen Ansprüche,
**dadurch gekennzeichnet, dass** sich an die Abführöffnung (14) ein Abführrohr (30) für das gasförmige Medium anschließt, welches sich abschnittsweise durch das Behältnis (6) hindurch erstreckt.

## Claims

1. Device for sterilizing vessels (10), comprising a treatment chamber (2) which is filled with a sterilizing gaseous medium (A) under predefined thermodynamic conditions, a container (6) which is filled with a liquid medium (B), which extends along a closed path, a plurality of holding devices (8) for holding the vessels, wherein the holding devices (8) are arranged such as to be able to rotate relative to at least one of the walls (18, 22, 23, 24) forming the treatment chamber (2), and a separating wall (12) which protrudes intro the liquid medium (B) located in the container (6) and is arranged such as to be able to rotate relative to the container (6), **characterized in that** a discharge opening (14), via which the gaseous medium (A) can be discharged, is provided between the treatment chamber (2) and the liquid medium (B) located in the container (6).

2. Device according to claim 1, **characterized in that** a buffer chamber (16) for the gaseous medium (A) is provided between the treatment chamber (2) and the liquid medium (B) located in the container (6).

3. Device according to claim 2, **characterized in that** the medium (A) in the buffer chamber (16) is at a lower pressure (p2) than in the treatment chamber (2).

4. Device (1) according to at least one of the preceding claims, **characterized in that** the separating wall is arranged on a cover (18) which covers the treatment chamber (2).

5. Device (1) according to at least one of the preceding claims, **characterized in that** the treatment chamber (2) is bounded by a stationary side wall (22) and a side wall (24) which can rotate relative to the stationary side wall (22).

6. Device (1) according to at least one of the preceding claims, **characterized in that** the device (1) comprises two containers (6) which are separated from one another and also two separating walls (12), which respectively protrude into the containers (6).

7. Device (1) according to at least one of the preceding claims, **characterized in that** the container (6) comprises a radially outer boundary wall (32) and a radially inner boundary wall (34), and the radially outer boundary wall (32) is lower than the radially inner boundary wall (34).

8. Device (1) according to at least one of the claims 2 and 3, **characterized in that** provided between the treatment chamber 2 and the buffer chamber (16) are at least two deflecting walls (34, 36) which deflect a flow direction of the gaseous medium (A) towards the discharge opening.

9. Device (1) according to claim 4, **characterized in that** the cover (18) is arranged in a rotatable manner.

10. Device (1) according to claims 9 and 4, **characterized in that** at least one nozzle (38) for cleaning the buffer chamber (16) is provided on the cover (18).

11. Device (1) according to at least one of the preceding claims, **characterized in that** at least one nozzle (39) for cleaning the container (6) is provided in the container (6).

12. Device (1) according to at least one of the preceding claims 2, 3 and 8, **characterized in that** at least one nozzle (37) arranged in a stationary manner is provided in the buffer chamber (16).

13. Device (1) according to at least one of the preceding claims, **characterized in that** the container (6) is arranged below the vessels (10) to be sterilized.

14. Device (1) according to at least one of the preceding claims, **characterized in that** the discharge opening (14) is arranged above a fill level (N) of the liquid medium (B).

15. Device (1) according to at least one of the preceding claims, **characterized in that** the discharge opening (14) is adjoined by a discharge pipe (30) for the gaseous medium, which extends partially through the container (6).

## Revendications

1. Installation de stérilisation de vases (10) avec un compartiment de traitement (2) rempli d'un fluide gazeux (A) de stérilisation dans des conditions thermodynamiques définies, avec un récipient (6) rempli d'un fluide liquide (B), qui s'étend le long d'un circuit fermé, avec une pluralité de dispositifs de maintien (8) destinés à maintenir les vases, lesdits dispositifs de maintien (8) étant disposés de manière pivotante par rapport à au moins une des parois (18, 22, 23, 24) formant le compartiment de traitement (2), avec une cloison de séparation (12) pénétrant dans le fluide liquide (B) contenu dans le récipient (6) et disposée de manière pivotante par rapport au récipient (6),
**caractérisée en ce qu'**
une ouverture d'évacuation (14) permettant d'évacuer le fluide gazeux (A) est prévue entre le compartiment de traitement (2) et le fluide liquide (B) contenu dans le réservoir (6).

2. Installation selon la revendication 1,
**caractérisée en ce qu'**
un compartiment tampon (16) pour le fluide gazeux (A) est prévu entre le compartiment de traitement (2) et le fluide liquide (B) contenu dans le réservoir (6).

3. Installation selon la revendication 2,
**caractérisée en ce que**
le fluide (A) est soumis dans le compartiment tampon (16) à une pression (p2) inférieure à celle du compartiment de traitement (2).

4. Installation (1) selon au moins une des revendications précédentes,
**caractérisée en ce que**
la cloison de séparation est disposée contre un couvercle (18) qui couvre le compartiment de traitement (2).

5. Installation (1) selon au moins une des revendications précédentes,
**caractérisée en ce que**
le compartiment de traitement (2) est délimité par une paroi latérale verticale (22) et par une paroi latérale (24) pivotante par rapport à la paroi latérale verticale (22).

6. Installation (1) selon au moins une des revendications précédentes,
**caractérisée en ce que**
ladite installation (1) comporte deux récipients (6) séparés l'un de l'autre ainsi que deux cloisons de séparation (12) pénétrant chacune dans les réservoirs (6).

7. Installation (1) selon au moins une des revendications précédentes,
**caractérisée en ce que**
le récipient (6) comporte une paroi de délimitation (32) radialement extérieure et une paroi de délimitation (34) radialement intérieure, et **en ce que** la paroi de délimitation (32) radialement extérieure est plus basse que la paroi de délimitation (34) radialement intérieure.

8. Installation (1) selon au moins une des revendications 2 et 3,
**caractérisée en ce qu'**
au moins deux parois de déviation (34, 36) sont prévues entre le compartiment de traitement (2) et le compartiment tampon (16), lesquelles dévient une direction d'écoulement du fluide gazeux (A) en direction de l'ouverture d'évacuation.

9. Installation (1) selon la revendication 4,
**caractérisée en ce que**
le couvercle (18) est disposé de manière pivotante.

10. Installation (1) selon les revendications 9 et 4,
**caractérisée en ce qu'**
au moins une buse (38) pour le nettoyage du compartiment tampon (16) est prévue contre le couvercle (18).

11. Installation (1) selon au moins une des revendications précédentes,
**caractérisée en ce qu'**
au moins une buse (39) pour le nettoyage du récipient (6) est prévue dans le récipient (6).

12. Installation (1) selon au moins une des revendications 2, 3 et 8,
**caractérisée en ce qu'**
au moins une buse (37) fixe est prévue dans le compartiment tampon (16).

13. Installation (1) selon au moins une des revendications précédentes,
**caractérisée en ce que**
le récipient (6) est disposé en dessous des vases (10) à stériliser.

14. Installation (1) selon au moins une des revendications précédentes,
**caractérisée en ce que**
l'ouverture d'évacuation (14) est disposée au-dessus d'un niveau de remplissage (N) du fluide liquide (B).

15. Installation (1) selon au moins une des revendications précédentes,
**caractérisée en ce qu'**
une conduite d'évacuation (30) pour le fluide gazeux est raccordée à l'ouverture d'évacuation (14), laquelle s'étend en partie au travers du récipient (6).
